# EUROPEAN PATENT SPECIFICATION

(11) **EP 1 297 805 B1**
(45) Date of publication and mention of the grant of the patent: **04.11.2009**
(21) Application number: 02256783.8
(22) Date of filing: 30.09.2002
(51) Int. Cl.: A61F 13/20, A61F 15/00

(54) **Individually packaged body fluid absorbent article**
Einzeln verpackter Körperflüssigkeitsabsorbierender Artikel
Article absorbant pour fluides corporels emballé individuellement

(30) Priority: 01.10.2001 JP 2001305254
(43) Date of publication of application: 02.04.2003
(73) Proprietor: UNI-CHARM CORPORATION, Shikokuchuo-shi, Ehime-ken (JP)
(72) Inventor: Suga, Ayami, c/o Technical Center, Mitoyo-gun, Kagawa-ken 769-1602 (JP)
(74) Representative: Parry, Christopher Stephen

(56) References cited:
- EP-A- 1 153 838
- CH-A- 414 453
- DE-U- 20 021 471
- DE-U- 20 021 474
- US-A- 3 643 661

## Description

### BACKGROUND OF THE INVENTION

### Field of the Invention

The present invention relates to an individually packaged body fluid absorbent article in which a body fluid absorbent article including an absorbent body to be inserted into the vagina of a wearer is individually packaged in a bag, more particularly, relates to an individually packaged body fluid absorbent article in which instructions for use can easily be noticed by a user upon use of the body fluid absorbent article.

### Description of the Related Art

As body fluid absorbent articles constructed to include an absorbent body to be inserted into the vagina and a string extending from a rear end of the absorbent body, so-called tampons have been widely known.

Such body fluid absorbent articles commercially sold today have generally been of two types, namely, a type of which the tampon is accommodated in an applicator for insertion of the tampon into the user's body, or a type of which the tampon is to be inserted into the user's body by fingers without using the applicator. In the former, the tampon combined with the applicator is individually packaged in a bag. In the latter, only the tampon is individually packaged in a bag.

Thereafter, a plurality of individually packaged body fluid absorbent articles are housed in a box, and put on the market. Commonly, a manual describing instructions for use of the tampon is folded, and housed in the box together with the plurality of individually packaged body fluid absorbent articles.

Such manual is often ignored by users accustomed to using the tampons, except for first-time users and users unaccustomed to using the tampons. However, it is important to read the manual before use in case of the body fluid absorbent article of which the absorbent body is to be inserted into the vagina. Commonly, the manual describes not only how to insert/remove the tampon but also a note for assuring the safety upon use.

For instance, the note describes that the string is attached to the absorbent body and the user should check the string so as to assure that the string will not be detached from the absorbent body by pulling it. In the case where the absorbent body is accommodated in the applicator, for instance, the note describes that the user should check whether the front end of the applicator is damaged (crushed or cracked) or not. In the case where the absorbent body is inserted into the vagina without checking the string, if the string cannot be found immediately (though it is out of the vagina in fact) by the user trying to remove a used absorbent body out of the vagina, for instance, the user may possibly be afraid that the body fluid absorbent article would have been a defective product having no string attached to the absorbent body, or that the string would have been detached from the absorbent body during use, and may possibly get upset. If the front end of the applicator should have been damaged, it is preferred not to use such product.

However, if the manual is enclosed in the box, as set forth above, the user may frequently ignore the manual because of getting tired of unfolding and reading it or neglect to read it. In addition, since the box houses a plurality of individually packaged body fluid absorbent articles, it is inconvenient to take the manual out of the box for reading every time when the individually packaged body fluid absorbent article is to be used. Furthermore, the manual may possibly be lost after the box has been opened and several body fluid absorbent articles have been used.

Prior art individually packaged body fluid absorbent articles are known from DE 20021471, which discloses a package that features an additional storage space for retaining a sweet or similar; CH 414453, which discloses individually foil wrapped absorbent articles that are each closed by a clamping element; DE 20021474, which discloses a package that may be provided with information printed thereon; EP 1153838, which discloses a package that may be provided with information printed thereon.

### SUMMARY OF THE INVENTION

The present invention has been worked out in view of the shortcoming in the prior art set forth above. It is therefore an object of the present invention to provide an individually packaged body fluid absorbent article, in which instructions for use can easily and certainly be noticed by a user every time when the individually packaged body fluid absorbent article is to be used.

According to a first aspect of the present invention, there is provided an individually packaged body fluid absorbent article comprising: a body fluid absorbent article including an absorbent body to be inserted into the vagina of a user; and a bag in which the body fluid absorbent article is individually packaged, wherein instructions for use of the body fluid absorbent article are indicated on the bag, characterised in that the bag has a separable section, defined by a tear line, which when separated exposes the absorbent article externally, and the instructions for use are indicated on at least one of exterior and interior surfaces of the separable section.

With such construction, since the instructions for use can easily catch user's attention every time when the individually packaged body fluid absorbent article is to be used, it hardly happens to use the article in an improper way or to neglect to check the article before use.

In one embodiment, the bag may be elongate, and the instructions for use may be longitudinally repeated at regular intervals on an exterior surface of the bag, over an entire length of the bag. In case where body fluid absorbent articles are sequentially packaged in a continuous strip-shaped packaging sheet and then cut to obtain individually packaged body fluid absorbent articles, if instructions for use are repeated at regular intervals on the continuous strip-shaped packaging sheet, the instructions for use can always be indicated on the exterior surface of the bag of each individually packaged body fluid absorbent article.

Preferably, the instructions for use are indicated on both the exterior and interior surfaces of the separable section so as to make the instructions for use more noticeable. In this case, it is also possible that instructions for use of which a user should be notified before opening the bag are indicated on the exterior surface of the separable section, and other instructions for use are indicated on the interior surface of the separable section.

Preferably, an instruction sheet, on which instructions for use of the body fluid absorbent article are indicated, is enclosed in the bag.

With such construction, since the instruction sheet comes out of the bag every time when the bag is opened, a can get into a habit of reading the instructions for use indicated on the instruction sheet.

In this case, if at least part of the instruction sheet is hydrophobic or water-repellent, the user's hand can easily be prevented from being stained with body fluids when a used absorbent body is wrapped in the instruction sheet for disposal. Here, it is also possible that the instruction sheet is in bag form for accommodating a used absorbent body.

### BRIEF DESCRIPTION OF THE DRAWINGS

The present invention will be understood more fully from the detailed description given hereinafter and from the accompanying drawings of the preferred embodiment of the present invention, which, however, should not be taken to be limitative to the invention, but are for explanation and understanding only.

In the drawings:
Fig. 1 is a perspective view showing an individually packaged body fluid absorbent article according to a first example that does not constitute an embodiment of the present invention;
Figs. 2A and 2B show instructions for use, which are printed on the exterior surface of a bag;
Fig. 3 is a perspective view showing how to open the bag;
Fig. 4 is a perspective view showing an individually packaged body fluid absorbent article according to a second example that does not constitute an embodiment of the present invention;
Fig. 5 is an enlarged view showing instructions for use on a bag;
Fig. 6 is a perspective view showing an individually packaged body fluid absorbent article according to a first embodiment of the present invention;
Fig. 7A is a top plan view of Fig. 6, and Fig. 7B shows a state where a bag is opened;
Fig. 8 is a perspective view showing an individually packaged body fluid absorbent article according to a second embodiment of the present invention;
Fig. 9 is a perspective view showing a state where a bag is opened;
Fig. 10 is a perspective view showing an individually packaged body fluid absorbent article with an instruction sheet ; and
Fig. 11A shows a state where an instruction sheet is rolled, and Fig. 11B shows a state where the instruction sheet is developed.

### DESCRIPTION OF THE PREFERRED EMBODIMENT

The present invention will be discussed hereinafter in detail in terms of the preferred embodiments according to he present invention with reference to the accompanying drawings. In the following description, numerous specific details are set forth in order to provide a thorough understanding of the present invention. It will be obvious, however, to those skilled in the art that the present invention may be practiced without these specific details. In other instance, well-known structures are not shown in detail in order to avoid unnecessary obscurity of the present invention.

The term "body fluid absorbent article" as used herein means an absorbent article of which an absorbent body is to be inserted into the vagina of a wearer for absorbing body fluids such as menstrual blood.

The term "individually packaged" as used herein means a state where each body fluid absorbent article is individually packaged in a bag. Here, it should be noted that an applicator as means for inserting the absorbent body into the vagina of a wearer may or may not be enclosed in the bag.

The term "bag" as used herein means a packaging material which is in the form of bag closed on all sides and accommodates a single body fluid absorbent article so as to keep the body fluid absorbent article clean until use.

The term "instructions for use" as used herein means any kind of statement concerning the usage of the body fluid absorbent article, such as how to take the absorbent article out of the bag, how to insert the absorbent body into the vagina of a wearer, how to dispose of a used absorbent article, how to check the absorbent article before use, and so on. The instructions for use may be described in any suitable manner such as letters (characters), illustration (explanatory drawing), or a combination thereof.

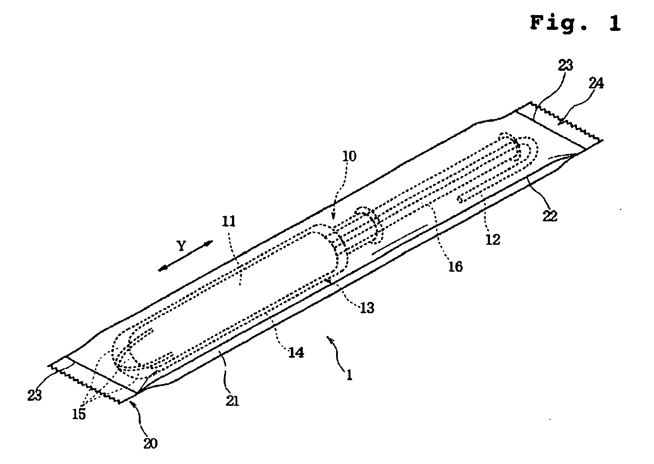

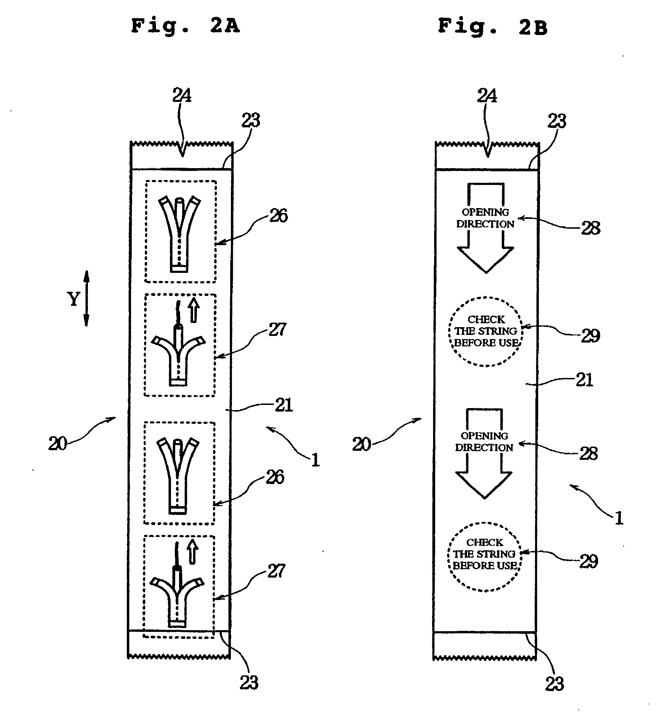

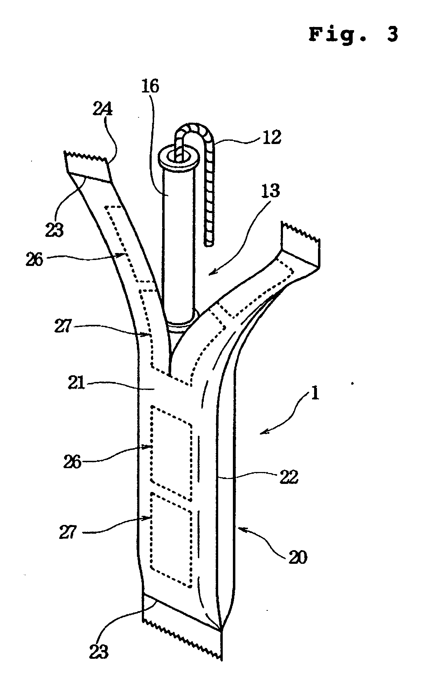

Fig. 1 is a perspective view showing an individually packaged body fluid absorbent article 1 according to a first example that does not constitute an embodiment of the present invention; Figs. 2A and 2B show instructions for use, which are printed on the exterior surface of a bag 20; and Fig. 3 is a perspective view showing how to open the bag 20.

The individually packaged body fluid absorbent article 1 shown in Figs. 1 to 3 is constructed by individually packaging a single body fluid absorbent article 10 in the bag 20. The body fluid absorbent article 10 comprises: a so-called tampon including an absorbent body 11 and a string extending from a rear end of the absorbent body 11; and an applicator 13 in which the tampon is housed.

The absorbent body 11 comprises: a core formed by compressing hydrophilic fibers such as cotton or rayon; and a liquid-permeable sheet such as nonwoven fabric in which the core is wrapped. When inserted into the woman's vagina during menstruation, the absorbent body 11 absorbs a menstrual blood to swell in the vagina. The string 12 is kept out of the vaginal opening while the absorbent body 11 is in the vagina, so that the absorbent body 11 can be taken out of the vagina after use by pulling the string 12.

The applicator 13 is formed of a synthetic resin material, and comprises: a front tubular member 14 housing the absorbent body 11; and a rear tubular member 16 slidably inserted into the front tubular member 14 so that the front end of the rear tubular member 16 confronts the rear end of the absorbent body 11. The front end of the front tubular member 14 is split to form a plurality of claws 15. The string 12 extending from the rear end of the absorbent body 11 passes through the rear tubular member 16 and projects rearwardly from the rear end of the rear tubular member 16.

When the body fluid absorbent article 10 is to be used, the front tubular member 14 of the applicator 13 is inserted into the vagina, and then, the rear tubular member 16 is pushed into the front tubular member 14. At this time, the absorbent body 11 is pushed out of the front tubular member 14 the rear tubular member 16, so that the claws 15 are deformed to open the front end of the front tubular member 14 and the absorbent body 11 is inserted into the vagina.

The bag 20 is formed of a packaging sheet 21. In the example shown, the packaging sheet 21 is a polyethylene film. However, it is also possible to employ a polypropylene film, paper, a laminate of polyethylene film and paper, or the like, in place of the polyethylene film.

The bag 20 is in the form of so-called pillow type packaging. In a production line, a continuous strip-shaped polyethylene film is first formed into a cylinder having a longitudinally extending seal 22. Then, after the body fluid absorbent article 10 is inserted in the cylinder, the cylinder is formed with transversely extending seals 23 and 23, in the front and the rear of the body fluid absorbent article 10. Finally, the cylinder is cut to obtain the individually packaged body fluid absorbent article 1.

At one end of the bag 20, there is formed a notch (i.e., V-shaped indentation) 24 for triggering tear of the bag 20. In order to make the tear progress in the longitudinal direction (Y-direction) of the bag 20, moreover, a line extending from the notch 24 in the longitudinal direction is perforated on the packaging sheet 21, or a resin film employed as the packaging sheet 21 is oriented in the longitudinal direction. When the bag 20 is pulled rightward and leftward from the notch 24, therefore, the packaging sheet 21 is longitudinally cut from the notch 24, as shown in Fig. 3, so that the bag 20 can be separated into right and left halves.

On the bag 20, instructions for use are indicated. In the example shown, the instructions for use are printed on one surface of the packaging sheet 21 forming the bag 20. Fig. 2A shows the exterior surface of the bag 20, as viewed from above, and Fig. 2B shows the exterior surface of the bag 20, as viewed from the opposite side.

On the exterior surface shown in Fig. 2A, there are indicated first instructions 26 and second instructions 27. On the exterior surface shown in Fig. 2B, there are indicated third instructions 28 and fourth instructions 29. The first instructions 26 and the second instructions 27 alternate with each other, and the sets of the first and second instructions 26 and 27 are repeated at regular intervals in the longitudinal direction (Y-direction), over the entire length of bag 20. Similarly, the third instructions 28 and the fourth instructions 29 alternate with each other, and the sets of the third and fourth instructions 28 and 29 are repeated at regular intervals in the longitudinal direction (Y-direction), over the entire length of bag 20.

The first instructions 26 are indicated by illustration only, and teach how to open (tear) the bag 20. The second instructions 27 are also indicated by illustration only, but teach how to check the body fluid absorbent article 10 before use. In the example shown, the second instructions 27 are intended to arouse a user to check the string 12 by pulling it before use. The third instructions 28 are indicated by an arrow and several letters, and teach a direction along which the bag 2 0 is to be torn. The fourth instructions 29 are indicated by several letters only, and arouse a user to check the string 12 before use.

Needless to say, instructions for use to be indicated on the bag 20 should not be limited thereto, but may include any kind of statement concerning the usage of the body fluid absorbent article 10. For instance, instructions for inserting the front tubular member 14 into the vagina and pushing the absorbent body 11 out of the front tubular member 14 with the rear tubular member 16 may be indicated by illustration or letters. In an alternative, instructions for assuring that nothing is wrong with the claws 15 at the front end of the front tubular member 14 may be indicated by illustration or letters. It is also possible to print such instructions on the packaging sheet 21 together with the first to fourth instructions 26 to 29.

Such instructions are printed on the packaging sheet 21 as patterns repeated in the longitudinal direction. The repetition interval of individual instructions is required to be smaller than the length of the bag 20. By printing the instructions as set forth above, it becomes possible to indicate at least one set of instructions on each bag 20 even if the packaging sheet 21 is continuously supplied for wrapping without positioning the instructions 26 to 29 with respect to each body fluid absorbent article 10 in the process of sequentially manufacturing individually packaged body fluid absorbent articles 1.

A plurality of individually packaged body fluid absorbent articles 1 are housed in a paper box for sale. Here, a manual describing instructions for use of the body fluid absorbent article 10 may be enclosed in the box, but since the instructions for use are indicated on each bag 20, it is not necessarily required to enclose such manual in the box.

When the individually packaged body fluid absorbent article 1 is taken out of the box for use, the instructions 26 to 29 indicated on the exterior surface of the bag 20 can easily catch user's attention. Therefore, the user hardly makes a mistake in opening operation of the bag 20, neglects to check the article before use, or makes a mistake in usage of the article.

In addition, since the first instructions 26 illustrate a torn bag with its illustrated torn end directed to the notch 24, the user can easily understand the position from which the bag 20 should be torn. On the other hand, since the letters of instructions 28 and 29 are written to be read in a state where the end having the notch 24 is directed upwardly, as shown in Fig. 2B, the user will begin to tear the bag 20 with the notch 24 directed upwardly. At this time, since the notch 24 is formed adjacent to the rear end of the body fluid absorbent article 10, i.e., in a rear section of the bag 20 accommodating the rear tubular member 16 of the applicator 13, the rear end of the rear tubular member 16 and the string 12 will appear at the beginning of tear of the bag 20 while being directed upwardly. Here, since the front tubular member 14 of the applicator 13 is still in the bag 20, the check of the string 12 can be performed in accordance with the second instructions 27 while holding the front tubular member 14 through the bag 20. Therefore, the front tubular member 14 can be kept clean.

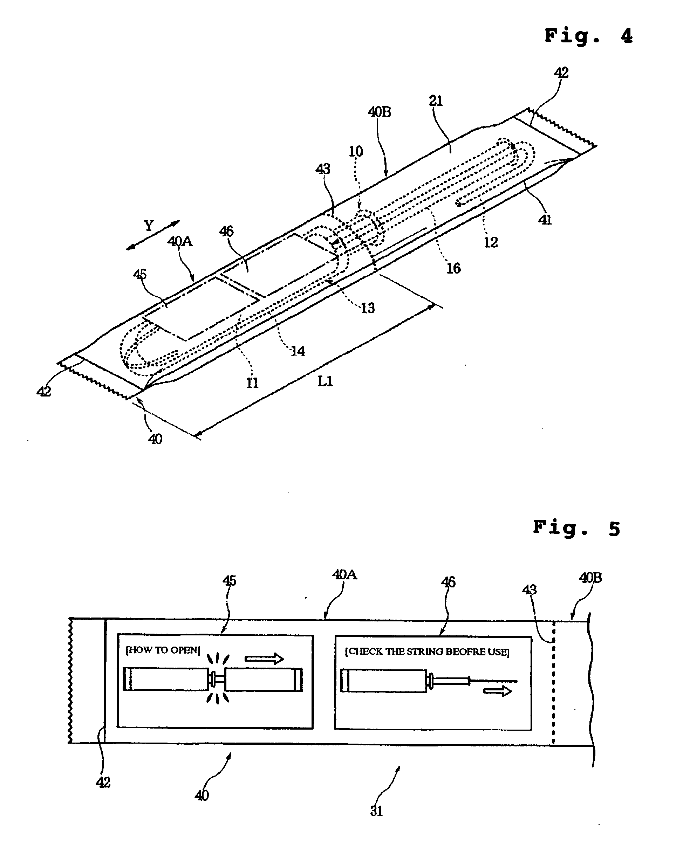

Fig. 4 is a perspective view showing an individually packaged body fluid absorbent article 31 according to a second example that does not constitute an embodiment of the present invention; and Fig. 5 is an enlarged view showing instructions for use indicated on a bag 40 of the individually packaged body fluid absorbent article 31.

The individually packaged body fluid absorbent article 31 is formed by packaging the body fluid absorbent article 10 in the bag 40. The body fluid absorbent article 10 is identical to that shown in Fig. 1, wherein the tampon comprising the absorbent body 11 and string 12 is housed in the applicator 13. Here, the detailed description of the portions having the same constructions as those of the first embodiment will be omitted by designating them by the common reference numerals.

The bag 40 is also formed of the packaging sheet 21 such as a polyethylene film. The bag 40 is formed such that the packaging sheet 21 is first formed into a cylinder which is sealed to have a longitudinally extending seal 41, and then, both ends of the cylinder, in which the body fluid absorbent article 10 is accommodated, are sealed to have transversely extending seals 42 and 42.

The bag 40 is provided at the center thereof with a tear section 43 for longitudinally separating the bag 40 into two. In the example shown, the tear section 43 is a line which is perforated on the packaging sheet 21 to extend continuously circumferentially of the bag 40. In an alternative, the tear section 43 may be formed such that a resin film in a transverse direction perpendicular the longitudinal direction (Y-direction) is employed as the packaging sheet 21 and the packaging sheet 21 is provided with a cut for triggering formation of a transversely extending cutoff line.

With the tear section 43, the bag 40 can be separated into a front section 40A and a rear section 40B. Here, the front tubular member 14 accommodating the absorbent body 11 of the body fluid absorbent article 10 is positioned in the front section 40A, and the rear tubular member 16 through which the string 12 passes is positioned in the rear section 40B.

In the example shown, instructions 45 and instructions 46 are printed on the exterior surface of the front section 40A of the bag 40.

As shown in Fig. 5 in an enlarged scale, the instructions 45 teach how to open (tear) the bag 40. The instructions 45 are indicated by illustration and letters, and teach a user that the bag 40 can be cut along the tear section 43 and separated into the front section 40A and the rear section 40B. The other instructions 46 are also indicated by illustration and letters, but intended to arouse a user to check the string 12 after the bag 40 is opened.

When the individually packaged body fluid absorbent article 31 is handled, since the letters of the instructions 45 and 46 are written as shown in Fig. 5, the front section 40A and the rear section 40B will be naturally held by the left hand and the right hand, respectively. Therefore, the front tubular member 14 can be held by the left hand through the front section 40A when the bag 40 is separated into two along the tear section 43. Then, the string 12 can be checked by pulling it in accordance with the instructions 46, while the front tubular member 14 is held by the left hand through the front section 40A. Therefore, the front tubular member 14 can be kept clean. Here, since the front tubular member 14 usually has a length of about 50 to 70 mm, the length L1 of the front section 40A is preferably in a range of about 40 to 80 mm so as to assure that the front tubular member 14 will be held through the front section 40A after the bag 40 is opened.

In the example shown, only the instructions 45 and 46 are indicated on the front section 40A accommodating the absorbent body 11 and the front tubular member 14. However, it is also possible to indicate instructions for use similar to or different from the instructions 45 and 46 on the exterior surface of the rear section 40B, in addition to the instructions 45 and 46 indicated on the front section 40A.

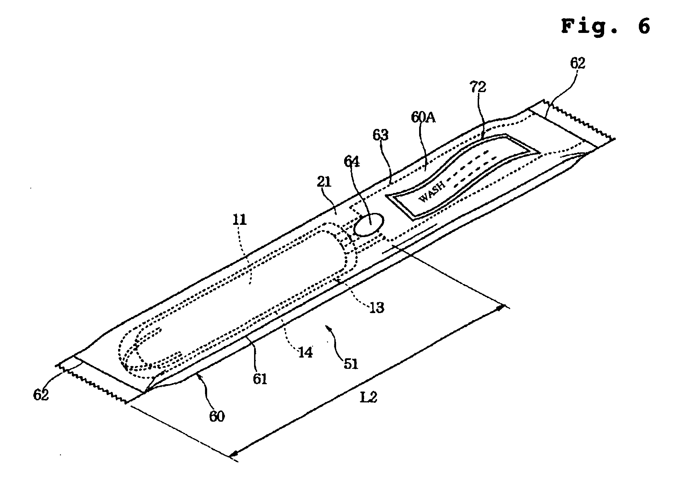

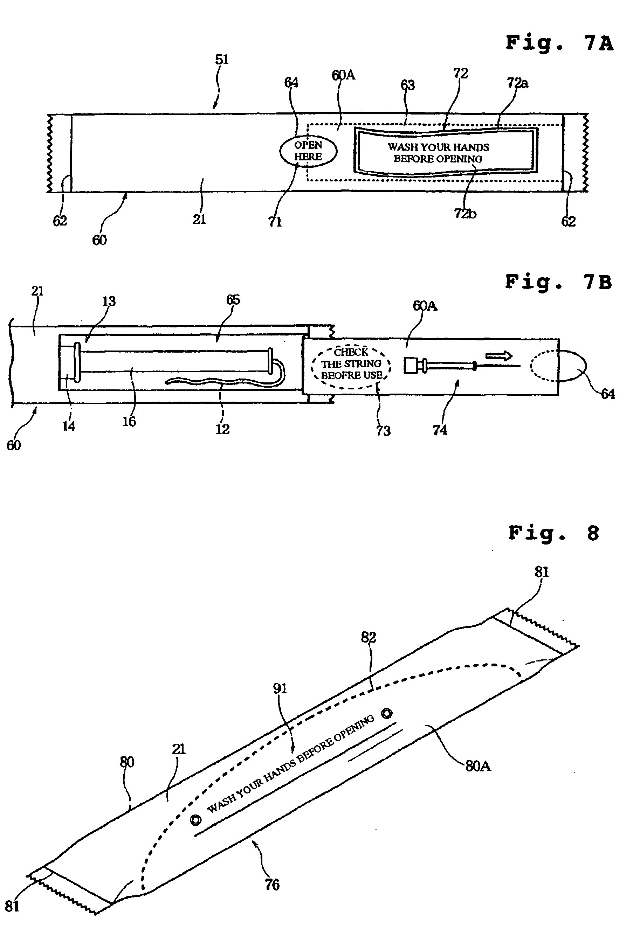

Fig. 6 is a perspective view showing an individually packaged body fluid absorbent article 51 according to a first embodiment of the present invention; Fig. 7A is a top plan view of Fig. 6; and Fig. 7B shows a state where a bag 60 of the individually packaged body fluid absorbent article 51 is opened.

The individually packaged body fluid absorbent article 51 is formed by packaging the body fluid absorbent article 10 in the bag 60. The body fluid absorbent article 10 is identical to that shown in Fig. 1, wherein the tampon comprising the absorbent body 11 and string 12 is housed in the applicator 13 comprising the front tubular member 14 and the rear tubular member 16. Here, the detailed description of the portions having the same constructions as those of the first embodiment will be omitted by designating them by the common reference numerals. The bag 60 is also formed of the packaging sheet 21 such as a polyethylene film. The bag 60 is sealed to have a longitudinally extending seal 61 and transversely extending seals 62 and 62.

In a rear section of the bag 60 accommodating the rear tubular member 16 of the applicator 13 and the string 12, there is formed a tear line 63 which is perforated on the packaging sheet 21. The rectangular section defined by the tear line 63 and the transversely extending seal 62 is a separable section 60A. In addition, a peelable seal 64 is adhered to the bag 60 to extend across the tear line 63. The seal 64 is firmly adhered to the separable section 60A, but weakly adhered to the remaining section of the bag 60 outside the tear line 63.

As shown in Fig. 7B, the separable section 60A can be separated from the remaining section of the bag 60 by peeling off the seal 64 and tearing the packaging sheet 21 along the tear line 63, to thereby form an opening 65 in the bag 60, through which the rear tubular member 16 of the applicator 13 and the string 12 can be exposed externally.

As shown in Fig. 7A, instructions 71 teaching that the bag 60 can be opened from here are printed in letters on the surface of the seal 64. On the exterior surface of the separable section 60A, printed are instructions 72 of which a user should be notified before opening the bag 60. The instructions 72 comprise an ornamental frame 72a and letters 72b written in the frame 72a. The instructions 72 describe "Wash your hands before opening."

On the interior surface of the separable section 60A, on the other hand, printed are instructions 73 and 74. When the separable section 60A is torn, the instructions 73 and 74 will appear, as shown in Fig. 7B. The instructions 73 are written in letters, describing that "Check the string before use." The other instructions 74 illustrate how to check the string 12. Of course, it is also possible to print other instructions for use on the interior surface of the separable section 60A by letters and/or illustration.

In the embodiment shown, too, since the letters 72b of the instructions 72 are written as shown in Fig. 7A, the front section of the bag 60 accommodating the front tubular member 14 will be naturally held by the left hand. When the separable section 60A is torn by the right hand while the front section is held by the left hand, the rear tubular member 16 and the string 12 will appear in the opening 65, as shown in Fig 7B. At the same time, the instructions 73 and 74 can catch user's attention. Therefore, a user will easily be aroused to check the string 12. In addition, since the string 12 can be checked by pulling it by the right hand while the front tubular member 14 is held through the bag 60 by the left hand, the front tubular member 14 can be kept clean. Here, the length L2 of the front section of the bag 60 not having the separable section 60A therein is preferably in a range of about 40 to 80 mm so as to assure that the front tubular member 14 will be held through the bag 60.

In the first embodiment, the separable section 60A of the bag 60 is formed by perforating the tear line 63 on the packaging sheet 21. That is, the separable section 60A is an integral part of the packaging sheet 21. However, the separable section should not be limited to such integral part of the packaging sheet 21. For instance, the separable section of the bag 60 may be provided such that an opening is preliminarily provided in the packaging sheet 21, and a small piece of sheet separate from the packaging sheet 21 is detachably adhered to the surface of the packaging sheet 21, around the opening, through a pressure sensitive adhesive.

In the first embodiment, the instructions for use are indicated on both the exterior and interior surfaces of the separable section 60A. However, it is, of course, possible to indicate the instructions for use on only one of the exterior and interior surfaces of the separable section 60A.

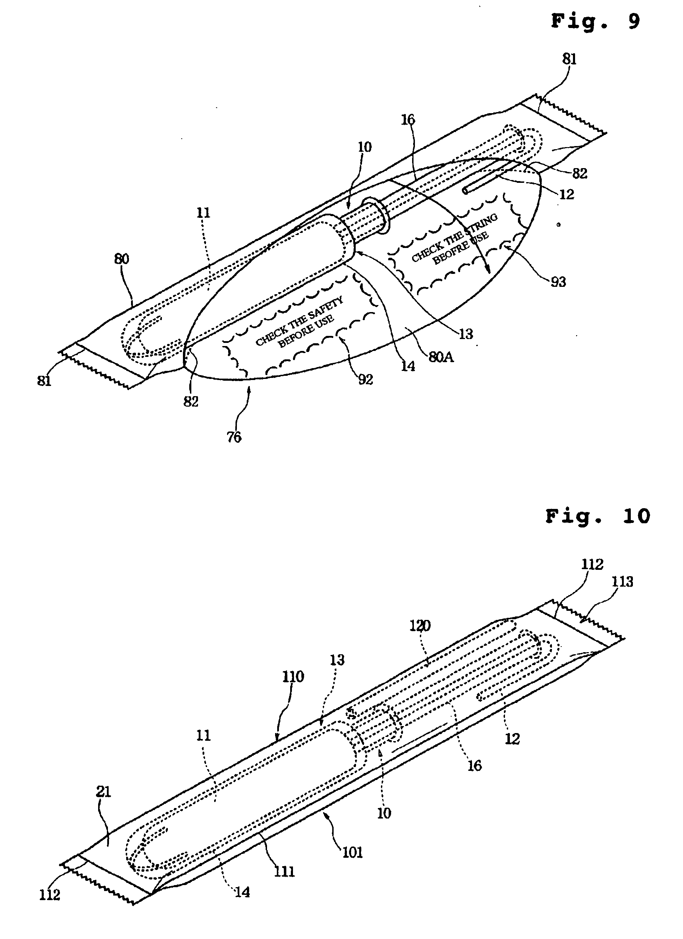

Fig. 8 is a perspective view showing an individually packaged body fluid absorbent article 76 according to a second embodiment of the present invention; and Fig. 9 is a perspective view showing a state where a bag 80 of the individually packaged body fluid absorbent article 76 is opened.

The individually packaged body fluid absorbent article 76 is formed by packaging the body fluid absorbent article 10 in the bag 80 formed of the packaging sheet 21. The body fluid absorbent article 10 is identical to that shown in Fig. 1, wherein the tampon comprising the absorbent body 11 and string 12 is housed in the applicator 13 comprising the front tubular member 14 and the rear tubular member 16, as shown in Fig. 9. Here, the detailed description of the portions having the same constructions as those of the first embodiment will be omitted by designating them by the common reference numerals.

The bag 80 is sealed to have a longitudinally extending seal (not shown) and transversely extending seals 81 and 81. Between the transversely extending seals 81 and 81 of the bag 80, a tear line 82 drawing an elliptical or oval shape is perforated on the packaging sheet 21. In the embodiment shown, the elliptical or oval section defined by the tear line 82 is a separable section 80A. As shown in Fig. 9, the separable section 80A can be separated from the remaining section of the bag 80 by tearing the packaging sheet 21 along the tear line 82, to thereby open the bag 80. The elliptical or oval separable section 80A may be separated either completely or partially from the remaining section of the bag 80.

On the exterior surface of the separable section 80A, as shown in Fig. 8, printed are instructions 91 of which a user should be notified before opening the bag 80. The instructions 91 describe "Wash your hands before opening." Of course, other instructions teaching that the bag 80 should be torn along the tear line 82 and so on may also be indicated thereon.

On the interior surface of the separable section 80A, on the other hand, printed are instructions 92 and 93. The instructions 92 and 93 will appear when the separable section 80A is separated, as shown in Fig. 9. The instructions 92 describe that "Check the safety before use" within an ornamental frame. The instructions 93 describe that "Check the string before use" within an ornamental frame. It is also possible to print illustrations for better understanding of such instructions.

In the second embodiment, too, the separable section may be formed of a material separate from the packaging sheet 21. For instance, the separable section of the bag 80 may be provided such that an opening is preliminarily provided in the packaging sheet 21, and a small piece of sheet separate from the packaging sheet 21 is detachably adhered to the surface of the packaging sheet 21, around the opening, through a pressure sensitive adhesive. It is also possible to indicate the instructions for use on only one of the exterior and interior surfaces of the separable section 80A.

In the foregoing embodiments, it is preferred that the packaging sheet 21 has a smooth surface and a superior wettability by ink, so as to make the printed instructions clear. In case where the instructions are printed on the interior surface of the bag, such as the instructions 73 and 74 of Fig. 7B or the instructions 92 and 93 of Fig. 9, on the other hand, the printed instructions are preferably coated with a varnish. With the varnish thus coated, the ink of the printed instructions can be preventing from adhering to the absorbent body 11, the string 12 or the applicator 13. Especially when the body fluid absorbent article 10 is of the type having no appllicator, i.e. the tampon comprising the absorbent body 11 and the string 12 is directly packaged in a bag, the varnishing is effective.

For instance, the varnish may contain a polyamide resin in a solvent consisting of three kinds of materials such as acetic acid esters, hydrocarbons and alcohol.

In the forgoing embodiments, it is also preferred that the illustrations and letters of the instructions are in contrasting colors with respect to the color of the packaging sheet 21. More preferably, the packaging sheet 21 is of white color or any pale color, and the instructions are printed in dark colors, such as black, red and green.

Fig. 10 is a perspective view showing an individually packaged body fluid absorbent article 101.

The individually packaged body fluid absorbent article 101 is formed by packaging the body fluid absorbent article 10 in a bag 110 formed of the packaging sheet 21. The body fluid absorbent article 10 is identical to that shown in Fig. 1, wherein the tampon comprising the absorbent body 11 and string 12 is housed in the applicator 13. Here, the detailed description of the portions having the same constructions as those of the first embodiment will be omitted by designating them by the common reference numerals.

The bag 110 is sealed to have a longitudinally extending seal 111 and transversely extending seals 112 and 112, and can be opened, for example, from a notch 113 provided in one end thereof, as shown in Fig. 3.

In the bag 110, an instruction sheet 120 is enclosed together with the body fluid absorbent article 10. This instruction sheet 120 may be paper. However, at least part of the instruction sheet 120 is preferably hydrophobic or water-repellent, considering that the instruction sheet 120 can also be used for covering user's fingers when a used absorbent article 11 is to be held by the fibers. For example, the instruction sheet 120 may be formed of a resin film, paper of which at least one surface is coated with a water-repellent resin, or paper of which at least one surface is applied a water-repellent agent by spraying or the like. In an alternative, a laminate of a hydrophilic sheet and a hydrophobic or water-repellent sheet may also be employed. For example, the laminate may be of a three-layer structure comprising top and bottom layers formed of a hydrophilic sheet such as paper and an intermediate layer formed of a hydrophobic sheet such as resin film. It is also possible to employ paper or nonwoven fabric containing hydrophobic fibers. As exemplified above, the instruction sheet 120 is preferably formed of a sheet material which can prevent a stain such as menstrual blood adhered to one surface thereof from oozing from the other surface thereof.

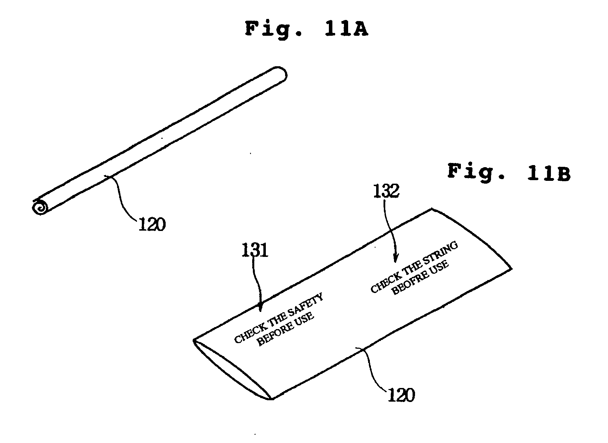

Fig. 11A shows a state where the instruction sheet 120 is rolled; and Fig. 11B shows a state where the instruction sheet 120 is developed. The instruction sheet 120 is formed of paper treated to be hydrophobic or water-repellent or a resin film, and is in the form of bag, as shown in Fig. 11B. After use, the tampon comprising the absorbent body 11 and the string 12 can be put in this bag for disposal. Therefore, the fingers can be prevented from being stained with body fluids such as menstrual blood

Moreover, instructions 131 and instructions 132 are printed on the instruction sheet 120. The instructions 131 describe that "Check the safety before use. "The instructions 132 describe that "Check the string before use." It is also possible to print illustrations for better understanding of such instructions.

The instruction sheet 120 comes out of the bag 110 every time when each individually packaged body fluid absorbent article 101 is opened. Therefore, a user can get into a habit of reading the instructions of the instruction sheet 120.

In an alternative, the instruction sheet 120 may be used as an auxiliary absorbent article. In this case, for example, the instruction sheet 120 is a laminate of a resin film and a thin absorbent sheet, wherein instructions for use are printed on the resin film. Upon use, the tampon is inserted into the vagina, and the instruction sheet 120 is put on an inner side of a crotch portion of an undergarment for absorbing a menstrual blood leaking out of the vaginal opening.

In the present invention, as has been described hereinabove, since the instructions for use can easily catch user's attention every time when the individually packaged body fluid absorbent article is to be used, it hardly happens to use the article in an improper way or to neglect to check the article before use.

## Claims

1. An individually packaged body fluid absorbent article comprising:
a body fluid absorbent article including an absorbent body to be inserted into the vagina of a user; and
a bag in which the body fluid absorbent article is individually packaged, wherein
instructions for use of the body fluid absorbent article are indicated on the bag, **characterised in that** the bag has a separable section, defined by a tear line, which when separated exposes the absorbent article externally, and the instructions for use are indicated on at least one of exterior and interior surfaces of the separable section.

2. The individually packaged body fluid absorbent article as set forth in claim 1, wherein the bag is elongate, and the instructions for use are longitudinally repeated at regular intervals on an exterior surface of the bag, over an entire length of the bag.

3. The individually packaged body fluid absorbent article as set forth in claim 1, wherein instructions for use of Which a user should be notified before opening the bag are indicated on the exterior surface of the separable section, and other instructions for use are indicated on the interior surface of the separable section.

4. An individually packaged body fluid absorbent article as set forth in claim 1, wherein an instructions sheet, on which instructions for use of the body fluid absorbent article are indicated, is enclosed in the bag.

5. The individually packaged body fluid absorbent article as set forth in claim 4, wherein at least part of the instruction sheet is hydrophobic or water-repellent,

6. The individually packaged body fluid absorbent article as set forth in claim 5, wherein the instruction sheet is in bag form for accommodating a used absorbent body.

## Patentansprüche

1. Einzeln verpackter Körperflüssigkeit absorbierender Artikel, umfassend:
einen Körperflüssigkeit absorbierenden Artikel einschließlich eines zum Einführen in die Vagina einer Benutzerin bestimmten absorbierenden Körpers; und
einen Beutel, in dem der Körperflüssigkeit absorbierende Artikel einzeln verpackt ist, wobei
Anweisungen zum Gebrauch des Körperflüssigkeit absorbierenden Artikels auf dem Beutel zu finden sind, **dadurch gekennzeichnet, dass** der Beutel einen von einer Reißlinie definierten abtrennbaren Abschnitt aufweist, der im abgetrennten Zustand den absorbierenden Artikel außen freilegt, und dass die Anweisungen zum Gebrauch auf mindestens einer der Außen- und Innenflächen des abtrennbaren Abschnitts zu finden sind.

2. Einzeln verpackter Körperflüssigkeit absorbierender Artikel nach Anspruch 1, wobei der Beutel länglich ist und die Anweisungen zum Gebrauch in regelmäßigen Abständen über die gesamte Länge des Beutels auf einer Außenfläche des Beutels wiederholt sind.

3. Einzeln verpackter Körperflüssigkeit absorbierender Artikel nach Anspruch 1, wobei Anweisungen zum Gebrauch, die der Benutzerin vor Öffnen des Beutels zur Kenntnis gebracht werden sollten, auf der Außenfläche des abnehmbaren Abschnitts und weitere Anweisungen zum Gebrauch auf der Innenfläche des abnehmbaren Abschnitts zu finden sind.

4. Einzeln verpackter Körperflüssigkeit absorbierender Artikel nach Anspruch 1, wobei ein Anweisungsblatt, auf welchem Anweisungen zum Gebrauch des Körperflüssigkeit absorbierenden Artikels zu finden sind, in den Beutel eingelegt sind.

5. Einzeln verpackter Körperflüssigkeit absorbierender Artikel nach Anspruch 4, wobei wenigstens ein Teil des Anweisungsblattes hydrophob oder wasserabstoßend ist.

6. Einzeln verpackter Körperflüssigkeit absorbierender Artikel nach Anspruch 5, wobei das Anweisungsblatt zur Aufnahme eines gebrauchten absorbierenden Körpers in Form eines Beutels vorliegt.

## Revendications

1. Article absorbant pour fluides corporels emballé individuellement comportant :
un article absorbant pour fluides corporels comprenant un corps absorbant destiné à être inséré dans le vagin d'une utilisatrice ; et
un sachet dans lequel l'article absorbant pour fluides corporels est emballé individuellement, dans lequel
des instructions d'emploi de l'article absorbant pour fluides corporels sont indiquées sur le sachet, **caractérisé en ce que** le sachet possède une section séparable, définie par une ligne à déchirer, qui quand elle est séparée expose l'article absorbant à l'extérieur, et les instructions d'emploi sont indiquées sur au moins l'une quelconque de la surface extérieure et de la surface intérieure de la section séparable.

2. Article absorbant pour fluides corporels emballé individuellement selon la revendication 1, dans lequel le sachet est allongé, et les instructions d'emploi sont répétées dans le sens longitudinal à intervalles réguliers sur une surface extérieure du sachet, sur toute une longueur du sachet.

3. Article absorbant pour fluides corporels emballé individuellement selon la revendication 1, dans lequel des instructions d'emploi qui doivent être communiquées à l'utilisatrice avant l'ouverture du sachet sont indiquées sur la surface extérieure de la section séparable, et d'autres instructions d'emploi sont indiquées sur la surface intérieure de la section séparable.

4. Article absorbant pour fluides corporels emballé individuellement selon la revendication 1, dans lequel une feuille d'instructions, sur laquelle des instructions d'emploi de l'article absorbant pour fluides corporels sont indiquées, est renfermée dans le sachet.

5. Article absorbant pour fluides corporels emballé individuellement selon la revendication 4, dans lequel au moins une partie de la feuille d'instructions est hydrophobe ou hydrofuge.

6. Article absorbant pour fluides corporels emballé individuellement selon la revendication 5, dans lequel la feuille d'instructions est sous la forme d'un sachet destiné à recueillir un corps absorbant usagé.
